# EUROPÄISCHE PATENTANMELDUNG

(11) **EP 3 690 876 A1**
(43) Veröffentlichungstag der Anmeldung: **05.08.2020**
(21) Anmeldenummer: 19154450.1
(22) Anmeldetag: 30.01.2019
(51) Int. Cl.: G10L 15/00, G10L 15/22

(54) **SYSTEM ZUR DURCHFÜHRUNG EINER MAGNETRESONANZTOMOGRAPHIE UND VERFAHREN ZUR STEUERUNG EINES MR SCANNERS**

(71) Anmelder: Siemens Healthcare GmbH, 91052 Erlangen (DE)
(72) Erfinder: SCHNEIDER, Rainer, Dr., 91054 Erlangen (DE); FRANGER, Dirk, 91052 Erlangen (DE)

(57) **Zusammenfassung**

Ein System zur Durchführung einer Magnetresonanztomographie, MR, umfasst einen MR Scanner, eine akustische Eingabevorrichtung zum Erfassen einer sprachlichen Äußerung und eine Steuerungsvorrichtung mit einem Eingang, welcher mit der Eingabevorrichtung und mit dem MR Scanner verbunden ist, und einem Ausgang, welcher mit dem MR Scanner verbunden ist. Die Steuerungsvorrichtung erzeugt aus der erfassten sprachlichen Äußerung einen Sprachdatenstrom und erstellt eine Befehlsbibliothek, welche eine Auswahl an Sprachbefehlen enthält, denen jeweils eine oder mehrere sprachliche Äußerungen zugeordnet sind, wobei die Auswahl an Sprachbefehlen in Abhängigkeit eines aktuellen Systemzustands des MR Scanners aus einer Befehlsdatenbank geladen wird. Die Steuerungsvorrichtung wendet einen Spracherkennungsalgorithmus auf den Sprachdatenstrom an, um zu ermittelt, ob dem Sprachdatenstrom eine in der Befehlsbibliothek enthaltene sprachliche Äußerung zuordenbar ist. Wenn dem Sprachdatenstrom eine in der Befehls-bibliothek enthaltene sprachliche Äußerung zuordenbar ist, wird die erfasste sprachliche Äußerung erkannt, ein der erkannten sprachlichen Äußerung zugeordneten Sprachbefehl aus der Befehlsbibliothek ermittelt und ein Steuerkommando zur Steuerung des MR Scanners gemäß dem Sprachbefehl erzeugt.

## Beschreibung

Die Erfindung betrifft ein System zur Durchführung einer Magnetresonanztomographie, kurz MR, und ein Verfahren zur Steuerung eines MR Scanners.

Die Magnetresonanztomographie, abgekürzt MRT oder MR, erleichtert als bildgebendes Verfahren die medizinische Diagnose in vielen Situationen. Zur Durchführung einer Magnetresonanztomographie werden MR-Scanner eingesetzt, welche eine hierin nicht näher erläuterte Spulenanordnung zur Erzeugung magnetischer Felder aufweisen, um basierend auf der Wechselwirkung der magnetischen Felder mit biologischen Bestandteilen des zu untersuchenden Körperbereichs des Patienten ein Bild zu erzeugen.

Vor und während der Durchführung einer MR Untersuchung eines Patienten sind üblicherweise verschiedene Einstellungen an dem MR Scanner vorzunehmen, wie beispielsweise eine Eingabe von Patientendaten, Einstellung verschiedener Scanparameter und dergleichen. Ferner muss der Patient in dem MR Scanner platziert werden. Diese Schritte werden typischerweise von medizinisch-technischem Personal durchgeführt, wobei die Vornahme der Einstellungen des Scanners in der Regel teils über ein am MR Scanner bereitgestelltes Interface und teils über einen sich in einem separaten Kontrollraum befindlichen Rechner erfolgt.

Um MR Systeme wirtschaftlich zu betreiben und den Komfort für Patienten während der Untersuchung zu verbessern, ist ein reibungsloser Arbeits- bzw. Verfahrensablauf wünschenswert. Die US 6 301 497 B1 beschreibt ein MR System, bei dem spezifische Funktionen des Scanners mit Hilfe einer Eingabevorrichtung in Form einer Sprachsteuerung aktiviert und deaktiviert werden können.

Vor diesem Hintergrund besteht ein Bedarf, ein Konzept zum effizienten Betreiben eines MR System bereitzustellen, z.B. ein Konzept, mit dem die Vornahme von Einstellungen des Systems erleichtert wird.

Diese Aufgabe wird durch die Merkmale der unabhängigen Ansprüche gelöst. Vorteilhafte Ausgestaltungen sind in den abhängigen Ansprüchen angegeben.

Gemäß einem ersten Aspekt der Erfindung ist ein Verfahren zur Steuerung eines MR Scanners vorgesehen. Erfindungsgemäß erfolgt ein Erfassen einer sprachlichen Äußerung mittels einer akustischen Eingabevorrichtung, z.B. durch Aufnehmen der sprachlichen Äußerung eines Bedieners des MR Scanners mittels eines Mikrophons. Aus der erfassten sprachlichen Äußerung wird ein Sprachdatenstrom erzeugt, beispielsweise über einen Analog-Digital-Wandler. Ferner wird ein aktueller Systemzustand des MR Scanners ermittelt. Der Systemzustand ist durch die Arbeitsschritte definiert, die der MR Scanner ausführt. Basierend darauf umfasst der Systemzustand Informationen darüber bzw. definiert, welche Operationen bzw. Arbeitsschritte der Scanner ausgehend von seinem momentanen Zustand potentiell durchführen könnte.

In einem weiteren Schritt des Verfahrens wird eine Befehlsbibliothek erstellt, welche eine Auswahl an Sprachbefehlen enthält, denen jeweils eine oder mehrere sprachliche Äußerungen zugeordnet sind. Ein Sprachbefehl, der auch als "intent" bezeichnet werden kann, kann als computerlesbarer Datensatz verstanden werden, der Information darüber enthält, durch welches Steuerkommando der MR Scanner betätigt werden soll. In der Befehlsbibliothek sind jeweils ein Sprachbefehl und ein oder mehrere Äußerungen, z.B. mehrere synonyme Begriffe oder Ausdrücke mit verschiedenen Wortreihenfolgen, einander zugeordnet. Erfindungsgemäß wird die Auswahl an Sprachbefehlen in Abhängigkeit des aktuellen Systemzustands des MR Scanners aus einer Befehlsdatenbank geladen. Das heißt, es wird überprüft, welcher Systemzustand beim MR Scanner vorliegt und je nach Systemzustand wird eine bestimmte Gruppe von Sprachbefehlen aus der Befehlsdatenbank in die temporäre Befehlsbibliothek geladen bzw. die Befehlsbibliothek wird temporär aus dieser Gruppe von Sprachbefehlen zusammengesetzt. Beispielsweise kann in der Befehlsdatenbank jedem Systemzustand eine vorbestimmte Gruppe von Sprachbefehlen zugeordnet sein. Insbesondere kann diese Gruppe nur aus Sprachbefehlen zusammengesetzt sein, die zur Erzeugung eines Steuerkommandos nutzbar sind, welches der Scanner in seinem Systemzustand tatsächlich ausführen kann.

In einem weiteren Schritt erfolgt ein Anwenden eines Spracherkennungsalgorithmus auf den Sprachdatenstrom, um zu ermittelt, ob dem Sprachdatenstrom eine in der Befehlsbibliothek enthaltene sprachliche Äußerung zuordenbar ist, und ein Erkennen der erfassten sprachlichen Äußerung, wenn dem Sprachdatenstrom eine in der Befehlsbibliothek enthaltene sprachliche Äußerung zuordenbar ist. Der Spracherkennungsalgorithmus kann insbesondere als Software realisiert sein.

Weiterhin erfolgt ein Ermitteln eines der erkannten sprachlichen Äußerung zugeordneten Sprachbefehls aus der Befehlsbibliothek. In diesem Schritt erfolgt also ein Auswählen desjenigen Sprachbefehls, der dem erkannten Ausdruck zugeordnet ist. Abschließend wird ein Steuerkommando zur Steuerung des MR Scanners gemäß dem ermittelten Sprachbefehl erzeugt. Das Steuerkommando kann insbesondere ein elektrisches Signal sein, das den MR Scanner zum Ausführen eines vorbestimmten Arbeitsschritts veranlasst, z.B. zum Erzeugen eines Magnetfeldes mit einer bestimmten Feldstärke.

Gemäß einem zweiten Aspekt der Erfindung ist ein nicht-flüchtiger, computerlesbarer Datenspeicher vorgesehen, welcher ein Softwareprogramm speichert, das dazu eingerichtet ist, einen Computer zur Durchführung der Schritte eines Verfahrens gemäß einem der voranstehenden Ansprüche zu veranlassen. Der nicht-flüchtige Datenspeicher kann insbesondere eine Festplatte, eine CD-ROM, eine DVD, eine Blu-Ray Disc, eine Diskette, einen Flash-Speicher oder dergleichen ausgebildet sein.

Gemäß einem dritten Aspekt der Erfindung ist ein System zur Durchführung einer Magnetresonanztomographie, kurz MR, vorgesehen. Das System umfasst einen MR Scanner zur Durchführung einer Scansequenz an einem Patienten, eine akustische Eingabevorrichtung zum Erfassen einer sprachlichen Äußerung und eine Steuerungsvorrichtung mit einem Eingang, welcher mit der Eingabevorrichtung und mit dem MR Scanner verbunden ist, einem Ausgang, welcher mit dem MR Scanner verbunden ist. Die Steuerungsvorrichtung weist also ein erstes Interface als Eingang und ein zweites Interface als Ausgang auf, wobei das erste und das zweite Interface jeweils zum drahtgebundenen oder drahtlosen Datenaustausch eingerichtet sind und beispielsweise als Bus-Schnittstelle, als WIFI Schnittstelle oder in ähnlicher Weise realisiert sein können. Die Eingabeeinrichtung und der MR Scanner stehen jeweils in Datenkommunikation mit dem Eingang der Steuerungsvorrichtung. Der MR Scanner ist zudem zum Datenaustausch mit dem Ausgang verbunden. Der Ein- und der Ausgang können hierbei durch physisch getrennte Anschlüsse bzw. Schnittstellen oder als ein gemeinsamer Anschluss realisiert sein.

Erfindungsgemäß ist die Steuerungsvorrichtung dazu eingerichtet aus der erfassten sprachlichen Äußerung einen Sprachdatenstrom zu erzeugen, einen aktuellen Systemzustand des MR Scanners zu ermitteln, eine Befehlsbibliothek zu erstellen, welche eine Auswahl an Sprachbefehlen enthält, denen jeweils eine oder mehrere sprachliche Äußerungen zugeordnet sind, wobei die Auswahl an Sprachbefehlen in Abhängigkeit des aktuellen Systemzustands des MR Scanners aus einer Befehlsdatenbank geladen wird, einen Spracherkennungsalgorithmus auf den Sprachdatenstrom anzuwenden, um zu ermitteln, ob dem Sprachdatenstrom eine in der Befehlsbibliothek enthaltene sprachliche Äußerung zuordenbar ist, die erfasste sprachliche Äußerung zu erkennen, wenn dem Sprachdatenstrom eine in der Befehlsbibliothek enthaltene sprachliche Äußerung zuordenbar ist, einen der erkannten sprachlichen Äußerung zugeordneten Sprachbefehl aus der Befehlsbibliothek zu ermitteln, und ein Steuerkommando zur Steuerung des MR Scanners gemäß dem Sprachbefehl zu erzeugen.

Allgemein kann das System gemäß dem dritten Aspekt der Erfindung zur Durchführung des Verfahrens gemäß dem ersten Aspekt der Erfindung vorgesehen und dazu eingerichtet sein. Die im Zusammenhang mit dem Verfahren gemäß dem ersten Aspekt der Erfindung offenbarten Merkmale und Vorteile sind daher auch für das System gemäß dem dritten Aspekt der Erfindung offenbart und umgekehrt.

Eine der Erfindung zugrundeliegende Idee besteht darin, eine Sprachsteuerung zum Steuern eines MR Scanners zu realisieren, indem dynamisch, abhängig von dem aktuellen Systemzustand bzw. dem Ist-Zustand, in welchem sich der MR Scanner befindet, eine Bibliothek mit für diesen Zustand zur Verfügung stehenden Sprachbefehlen generiert wird. Dies bietet den Vorteil, dass die Anzahl an Äußerungen, die zusammen mit dem Sprachdatenstrom dem Spracherkennungsalgorithmus als Eingangsgrößen bzw. Eingangsdaten zugeführt werden, gegenüber der gesamten Anzahl an Äußerungen, die in der Sprachbefehlsdatenbank enthalten sind, verringert wird. Auf diese Weise wird einerseits die Rechenleistung, die zur Ausführung des Spracherkennungsalgorithmus notwendig ist, vorteilhaft verringert. Ein weiterer Vorteil liegt darin, dass durch die verringerte Anzahl der Äußerungen in der Bibliothek die Zuverlässigkeit der Spracherkennung verbessert wird. Dies ist insbesondere deshalb von Vorteil, da MR Scanner typischerweise laute Geräusche erzeugen, die die Erkennung von Sprachbefehlen erschwert. Durch die dynamische Reduzierung der Auswahlmöglichkeiten wird die Sprachsteuerung robuster und die Fehleranfälligkeit wird verringert. Auf diese Weise wird der Arbeitsablauf am MR Scanner beschleunigt, da das Bedienpersonal den Scanner durch Sprachbefehle effizient steuern kann. Die Spracherkennung bietet zudem den Vorteil, dass sie ohne physische, insbesondere ohne manuelle Interaktion mit der Eingabeeinrichtung erfolgt, wodurch hygienische Vorteile erzielt werden.

Gemäß einer Ausführungsform des Verfahrens ist vorgesehen, dass, wenn dem Sprachdatenstrom keine in der Befehlsbibliothek enthaltene sprachliche Äußerung zuordenbar ist, der Sprachdatenstrom einem internetbasierten Online-Spracherkennungsmodul zugeführt wird, wobei das Online-Spracherkennungsmodul eine durch maschinelles Lernen trainierten Erkennungsfunktion auf den Eingangsdatenstrom anwendet und eine erkannte sprachliche Äußerung als Ausgangsdatenstrom bereitstellt. Demnach wird, falls der Spracherkennungsalgorithmus dem Sprachdatenstrom keine in der Befehlsbibliothek enthaltene sprachliche Äußerung zuordnen kann, ein durch maschinelles Lernen trainierter, computer-implementierter Algorithmus verwendet. Dieses Online-Spracherkennungsmodul kann über Internet beispielsweise Daten von einer Vielzahl von MR Systemen erhalten. Dadurch wird die anwendungsspezifische Zuverlässigkeit der Spracherkennung weiter verbessert.

Wenn mittels des Online-Spracherkennungsmodul eine bestimmte sprachliche Äußerung erkannt wurde, kann optional überprüft werden, ob die erkannte sprachliche Äußerung in der Befehlsbibliothek einem Sprachbefehl zugeordnet ist. Wenn dies der Fall ist, kann das Verfahren, wie oben beschrieben fortgesetzt werden. Dadurch wird die Bedienung des MR Scanners erleichtert, da die versehentliche Eingabe falscher Sprachbefehle vermieden wird.

Als aktueller Systemzustand des MR Scanners kann insbesondere einer oder mehrerer der folgenden Zustände ermittelt werden:
a) der MR Scanner befindet sich in einem Standby-Betrieb;
b) der MR Scanner führt eine vorbestimmte Scansequenz durch;
c) der MR Scanner befindet sich in einem Pausierzustand, wobei die Durchführung einer vorbestimmten Scansequenz unterbrochen ist;
d) der MR Scanner ist in einem Vorbereitungszustand zur Durchführung einer vorbestimmten Scansequenz. Wenn beispielsweise ermittelt wird, dass der MR Scanner sich aktuell im Zustand b) befindet, kann die Sprachbefehlsbibliothek z.B. so zusammengestellt werden, dass diese nur Sprachbefehle enthält, gemäß denen ein Steuerkommando erzeugt wird, welches den MR Scanner dazu veranlasst, die Scansequenz zu stoppen. In Zustand b) kann der Systemzustand ferner durch die Art der Scansequenz, die gerade durchgeführt wird, festgelegt sein. Hierdurch kann beispielsweise festgelegt sein, ob bestimmte Einstellungsparameter der Sequenz gelesen/verändert werden können. Ausgehend von dem Systemzustand ergibt sich beispielsweise auch, welche Sequenzen geöffnet, verändert und gestartet werden können.

Gemäß einer Ausführungsform des Datenspeichers kann vorgesehen sein, dass die Befehlsdatenbank auf dem Datenspeicher gespeichert ist. Dies bietet den Vorteil, dass ein besonders schneller Zugriff auf die Sprachbefehle und die zugehörigen Ausdrücke möglich ist, was die Erkennung weiter beschleunigt und erleichtert.

Gemäß einer Ausführungsform des Systems weist die Eingabevorrichtung ein Mikrophon auf.

Gemäß einer weiteren Ausführungsform des Systems ist vorgesehen, dass die Eingabevorrichtung portabel ist und einen Sender zur drahtlosen Datenübertragung aufweist, wobei der Eingang der Steuerungsvorrichtung einen Empfänger zur drahtlosen Datenübertragung aufweist, der mit dem Sender der Eingabevorrichtung kommuniziert. Dies bietet den Vorteil, dass die Eingabevorrichtung räumlich getrennt von der Steuerungsvorrichtung angeordnet werden kann, was die Arbeitsabläufe am MR Scanner weiter erleichtert.

Gemäß einer weiteren Ausführungsform weist die Steuerungsvorrichtung den nicht-flüchtigen Datenspeicher gemäß dem zweiten Aspekt der Erfindung und einen Prozessor zum Lesen des Datenspeichers auf.

Unter einem "Prozessor" ist eine elektronische Schaltung zur computerbasierten Datenverarbeitung zu verstehen, beispielsweise eine CPU. Es kann sich um die CPU eines Computers handeln oder um einen Mikroprozessor eines Mikrochips. Ferner kann der Prozessor auch als FPGA oder als ASIC realisiert sein.

Die Erfindung wird nachfolgend anhand von Ausführungsbeispielen mit Hilfe der Figuren näher erläutert. Es zeigen:
Figur 1 eine schematische Darstellung eines Systems zur Durchführung eine Magnetresonanztomographie gemäß einem Ausführungsbeispiel der Erfindung; und
Figur 2 ein Flussdiagramm eines Verfahrens zur Steuerung eines MR Scanners gemäß einem Ausführungsbeispiel der Erfindung.

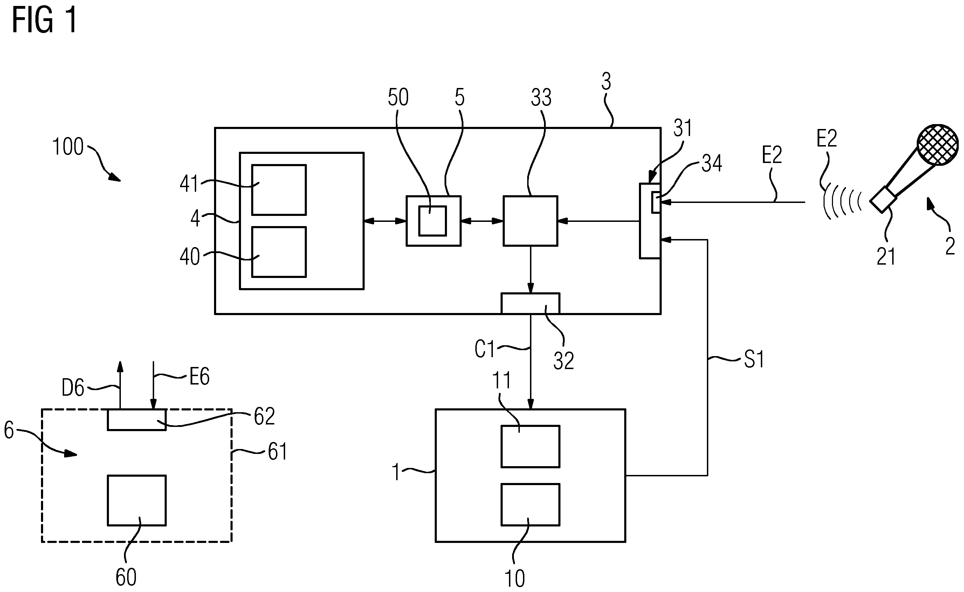

Figur 1 zeigt schematisch eine funktionale Blockdarstellung eines Systems 100 zur Durchführung eine Magnetresonanztomographie, MR. Das System 100 umfasst einen MR Scanner 1, eine akustische Eingabevorrichtung 2 und eine Steuerungsvorrichtung 3. In Figur 1 ist außerdem ein Online-Spracherkennungsmodul 6 als Block dargestellt, welches mit dem System 100 verbindbar ist.

Der MR Scanner 1 ist zur Durchführung einer Scansequenz an einem Patienten eingerichtet und wird hierin nicht näher erläutert. Insbesondere kann der MR Scanner 1 einen Erzeugerkreis 10 zur eines statischen Magnetfelds und zur Erzeugung von magnetischen Wechselfeldern im Radiofrequenzbereich, mit denen bestimmte Atomkerne im Körper des Patienten resonant angeregt werden, und einen Empfängerkreis 11, in welchen durch die Anregung der Atomkerne im Körper des Patienten ein elektrisches Signal induzierbar ist.

Die akustische Eingabevorrichtung 2 dient zum Aufnehmen oder Erfassen einer sprachlichen Äußerung E2, also zum Aufnehmen gesprochener Laute, die von einer Bedienperson erzeugt werden. Die Eingabevorrichtung 2 kann beispielsweise als Mikrophon realisiert sein. Die Eingabevorrichtung 2 kann beispielsweise an dem MR Scanner 1 oder an anderer Stelle, wie in einem Bedienraum, stationär angeordnet sein. Alternativ kann die Eingabevorrichtung 2 auch portabel realisiert sein, z.B. als Mikrophon eines Headsets, das von der Bedienperson mitführbar ist. In diesem Fall weist die Eingabevorrichtung 2 vorteilhaft einen Sender 21 zur drahtlosen Datenübertragung auf.

Die Steuerungsvorrichtung 3 weist einen Eingang 31 zum Empfang von Signalen und einen Ausgang 32 zum Bereitstellen von Signalen auf. Die Steuerungsvorrichtung 3 ist allgemein zur Durchführung von Datenverarbeitungsprozessen und zur Erzeugung elektrischer Signale eingerichtet. Hierzu kann die Steuerungsvorrichtung 3 einen Prozessor 33, z.B. in Form einer CPU oder dergleichen, und einen Datenspeicher 4, insbesondere einen durch den Prozessor 33 lesbaren, nicht-flüchtigen Datenspeicher wie eine Festplatte, eine CD-ROM, eine DVD, eine Blu-Ray Disc, eine Diskette, einen Flash-Speicher oder dergleichen, aufweisen. Auf dem Datenspeicher 4 kann allgemein Software 40, 41 gespeichert sein, die dazu eingerichtet ist, den Prozessor 33 zur Durchführung der Schritte eines Verfahrens zu veranlassen.
Wie in Figur 1 schematisch dargestellt ist, ist der Eingang 31 der Steuerungsvorrichtung 3 mit der Eingabevor-richtung 2 und mit dem MR Scanner 1 verbunden. Der Eingang 31 kann zur drahtlosen oder zur drahtgebundenen Datenkommunikation eingerichtet sein. Beispielsweise kann der Eingang 31 einen Bus-Anschluss aufweisen. Alternativ oder zusätzlich zu einem drahtgebundenen Anschluss kann der Eingang 31 auch eine Schnittstelle, z.B. einen Empfänger 34 drahtlosen Datenübertragung aufweisen. Beispielsweise kann, wie in Figur 1 dargestellt, der Empfänger 34 mit dem Sender 21 der Eingabevorrichtung 2 in Datenkommunikation stehen. Als Empfänger 34 kann beispielsweise eine WIFI Schnittstelle, eine Bluetooth-Schnittstelle oder dergleichen vorgesehen sein.

Der Ausgang 32 der Steuerungsvorrichtung 3 ist mit dem MR Scanner 1 verbunden. Der Ausgang 32 kann zur drahtlosen oder zur drahtgebundenen Datenkommunikation eingerichtet sein. Beispielsweise kann der Ausgang 32 einen Bus-Anschluss aufweisen. Alternativ oder zusätzlich zu einem drahtgebundenen Anschluss kann der Ausgang 32 auch eine Schnittstelle zur drahtlosen Datenübertragung aufweisen, beispielsweise eine WIFI Schnittstelle, eine Bluetooth-Schnittstelle oder dergleichen.

Die Steuerungsvorrichtung 3 ist dazu eingerichtet, ein Steuerkommando C1 zur Steuerung des MR Scanners 1 zu erzeugen und an dem Ausgang 32 bereitzustellen. Das Steuerkommando C1 veranlasst den MR Scanner 1 zur Durchführung eines bestimmten Arbeitsschritts oder einer Sequenz von Schritten, beispielsweise zur Durchführung einer bestimmten Scansequenz mit einer bestimmten Anregung von Magnetfeldern durch den Erzeugerkreis 10.

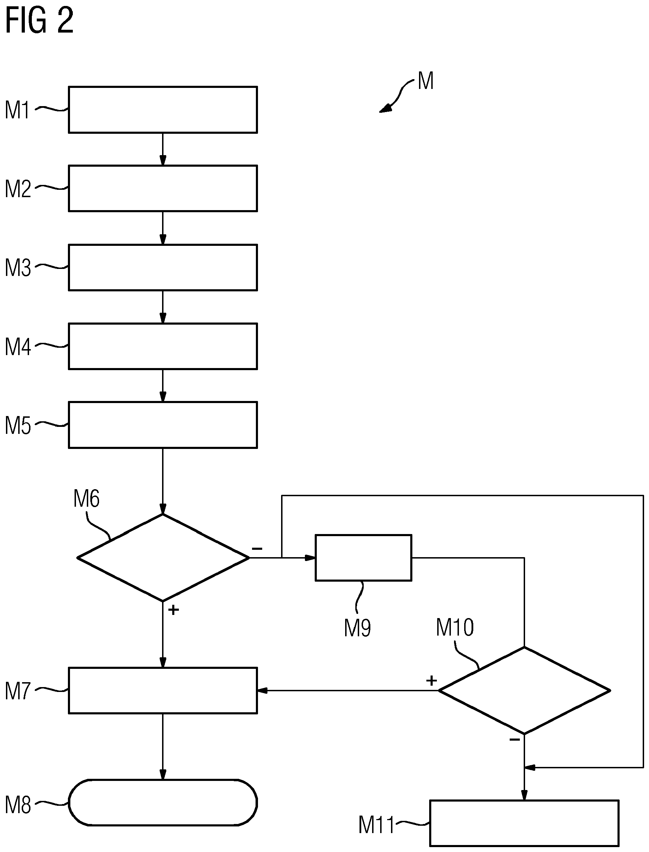

Die Steuerung des MR Scanners 1 erfolgt in dem in Figur 1 beispielhaft dargestellten System 100 durch ein Verfahren M, welches in Figur 2 beispielhaft als Flussdiagramm dargestellt ist. Allgemein ist hierbei vorgesehen, dass die Bedienperson (nicht dargestellt), welche den MR Scanner 1 bedient, ein Kommando stimmlich bzw. sprachlich äußert, z.B. indem sie einen Satz wie "Starte Scansequenz X" spricht, die Eingabevorrichtung 2 diese sprachliche Äußerung E2 erfasst und die Steuerungsvorrichtung 3 die erfasste sprachliche Äußerung E2 analysiert und ein entsprechendes Steuerkommando C1 zur Betätigung des MR Scanners 1 erzeugt. Ein Vorteil dieses Vorgehens ist, dass die Bedienperson während des Sprechens auch andere Aufgaben erledigen kann, z.B. sich mit der Vorbereitung des Patienten befassen kann. Dies beschleunigt vorteilhaft die Arbeitsabläufe. Ferner kann der MR Scanner 1 dadurch zumindest teilweise "berührungslos" gesteuert werden, wodurch die Hygiene am MR Scanner 1 verbessert wird.

Wie in Figur 2 durch den Block M1 dargestellt, erfolgt zunächst ein Erfassen der sprachlichen Äußerung E2 mittels der Eingabevorrichtung 2. Diese sprachliche Äußerung E2 wird der Steuerungsvorrichtung 3 am Eingang zur Verfügung gestellt und die Steuerungsvorrichtung 3 erzeugt aus der erfassten sprachlichen Äußerung E2 einen Sprachdatenstrom (Block M3). Hierzu kann beispielsweise ein Analog-Digital-Wandler (nicht dargestellt) vorgesehen sein. Das Erzeugen des Sprachdatenstroms kann optional einen Schritt umfassen, in welchem ein Anfang und ein Ende der gesprochenen Äußerung innerhalb einer gesprochenen Sequenz von Äußerungen detektiert werden. Dies kann beispielsweise durch eine Software realisiert werden, die auf dem Datenspeicher 4 abgelegt ist und den Prozessor 33 zur Durchführung dieses Schritts veranlasst. In diesem Fall wird außerdem die erfasste sprachliche Äußerung E2 als Sprachdatenstrom aus dem gesamten Datenstrom, der die Sequenz von Äußerungen enthält, extrahiert.

Wie in Figur 2 durch Block M3 symbolisch dargestellt, erfolgt in einem weiteren Schritt ein Ermitteln M3 eines aktuellen Systemzustands S1 des MR Scanners 1. Der Systemzustand S1 des MR Scanners S1 kann beispielsweise durch einen Standby-Betrieb des MR Scanners 1 gegeben sein oder dadurch, dass der MR Scanner 1 gerade eine vorbestimmte Scansequenz durchführt oder sich in einem Vorbereitungszustand zur Durchführung einer vorbestimmten Scansequenz befindet. Allgemein ist der Systemzustand S1 durch einen jeweiligen Arbeitsschritt oder eine Abfolge bzw. Sequenz von Arbeitsschritten festgelegt, die der MR Scanner gerade ausführt. Daraus ergibt sich, welche weiteren Arbeitsschritte der Scanner 1 potenziell durchführen könnte und damit, wie er betätigbar ist. Beispielsweise kann der Systemzustand als Eingangsgröße einem Look-up-Table zugeführt werden, in welchem die für verschiedene Systemzustände notwendigen Informationen für die Ansteuerung des MR Scanners 1 enthalten sind. Dieser Systemzustand S1 stellt der MR Scanner 1 am Eingang 31 der Steuerungsvorrichtung 3 bereit, z.B. als Datensignal.

In einem weiteren Schritt M4 wird eine Befehlsbibliothek 50 erstellt, welche eine Auswahl an Sprachbefehlen enthält, denen jeweils eine oder mehrere sprachliche Äußerungen zugeordnet sind, wobei die Auswahl an Sprachbefehlen in Abhängigkeit des aktuellen Systemzustands S1 des MR Scanners aus einer Befehlsdatenbank 40 geladen wird. Die Befehlsbibliothek 50 wird temporär für einen jeweiligen Systemzustand S1 generiert und kann beispielsweise als temporäre Datei in einen Arbeitsspeicher 5 der Steuerungsvorrichtung 3 geladen werden. Der Inhalt der Befehlsbibliothek 50, also die einzelnen Datensätze in denen jeweils ein Sprachbefehl mit einer oder mehrerer sprachlichen Äußerungen verknüpft ist, wird aus einer Befehlsdatenbank 40 geladen, die beispielsweise auf dem Datenspeicher 4 abgelegt sein kann. Welche Datensätze aus der Befehlsdatenbank 40 in die Befehlsbibliothek 50 geladen werden, hängt vom Systemzustand S1 des MR Scanners 1 ab. Beispielsweise kann der MR Scanner 1 bei der Durchführung einer bestimmten Scansequenz lediglich bestimmte andere oder weitere Arbeitsschritte ausführen. Diese Information ist in der Befehlsdatenbank 40 zusammen mit einem Sprachbefehl, welcher die Erzeugung eines dem Arbeitsschritt entsprechenden Steuerkommandos C1 bewirkt, hinterlegt. In Schritt M4 wird also eine Auswahl an Sprachbefehlen für den jeweils vorliegenden Systemzustand S1 des MR Scanners aus einer Befehlsdatenbank 40 geladen.

In Schritt M5 erfolgt ein Anwenden M5 eines Spracherkennungsalgorithmus auf den Sprachdatenstrom, um zu ermittelt, ob dem Sprachdatenstrom eine in der Befehlsbibliothek 50 enthaltene sprachliche Äußerung zuordenbar ist. Der Spracherkennungsalgorithmus kann beispielsweise als Software 41 auf dem Datenspeicher 4 enthalten sein. Wenn dem Sprachdatenstrom eine in der Befehlsbibliothek 50 enthaltene sprachliche Äußerung zuordenbar ist, wird in Schritt M6 die erfasste sprachliche Äußerung E2 erkannt, wie dies in Schritt M6 als "+" symbolisch dargestellt ist. In den Schritten M5 und M6 wird also entschlüsselt, ob die erfasste sprachliche Äußerung E2, die in den Sprachdatenstrom umgewandelt wurde, zu einer in der Befehlsbibliothek 50 enthaltenen Äußerung passt. Dies funktioniert bei dem beschriebenen Verfahren M besonders zuverlässig, da lediglich eine begrenzte Anzahl an Äußerungen in der Befehlsbibliothek 50 enthalten sind, nämlich nur diejenigen, die zu für den jeweiligen Systemzustand S1 des Scanners 1 möglichen Sprachbefehlen gehören. Damit wird die Zuverlässigkeit der Spracherkennung verbessert.

In Schritt M7, welcher nur durchgeführt wird, wenn in Schritt M6 die erfasste sprachliche Äußerung E2 erkannt wurde (Symbol "+" in Figur 2), wird ein der erkannten sprachlichen Äußerung zugeordneter Sprachbefehl aus der Befehlsbibliothek 50 ermittelt. Dieser kann beispielsweise in Form einer Eingangsvariable einem Erzeugungsmodul einer Software, die beispielsweise auf dem Datenspeicher 4 abgelegt ist, zugeführt werden, welche den Prozessor 33 dann zum Erzeugen (Block M8) eines Steuerkommandos C1 zur Steuerung des MR Scanners gemäß dem Sprachbefehl veranlasst.

Wenn in Schritt M6 die erfasste sprachliche Äußerung E2 nicht erkannt wurde (Symbol "-" in Figur 2), weil dem Sprachdatenstrom keine in der Befehlsbibliothek 50 enthaltene sprachliche Äußerung zuordenbar ist, kann das Verfahren M beendet werden. Optional kann zusätzlich ein weiterer Schritt M9 durchgeführt werden, wie dies in Figur 2 dargestellt ist. Hierbei wird der Sprachdatenstrom einem internetbasierten Online-Spracherkennungsmodul 6 als Eingangsdatenstrom E6 zugeführt. Das Online-Spracherkennungsmodul 6 kann auf einem Server 61 abgelegt sein, mit dem die Steuerungsvorrichtung 3 über eine Internetverbindung in Datenaustausch treten kann.

Das Online-Spracherkennungsmodul 6 kann eine durch maschinelles Lernen trainierte Erkennungsfunktion 60 als Software aufweisen, welche auf den Eingangsdatenstrom E6 anwendet wird und eine erkannte sprachliche Äußerung als Ausgangsdatenstrom D6 an einer Schnittstelle 62 des Servers 61 bereitstellt. Dieser Ausgangsdatenstrom D6 kann beispielsweise über den Eingang 31 der Steuerungsvorrichtung 3 zugeführt werden. In einem optionalen weiteren Schritt M10 wird überprüft, ob die erkannte sprachliche Äußerung in der Befehlsbibliothek 50 einem Sprachbefehl zugeordnet ist, wie dies oben bereits anhand von Schritt M6 beschrieben wurde. Wenn in Schritt M10 die erfasste sprachliche Äußerung E2 erkannt wurde (Symbol "+" in Figur 2), werden die Schritte M 7 und M8, wie oben beschrieben durchgeführt. Wenn die sprachliche Äußerung E2 in Schritt M10 nicht erkannt wurde, wie dies in Figur 2 durch das Symbol "-" dargestellt ist, ist das Verfahren beendet (Block M11).

In den Schritten M3 bis M8, ohne Durchführung der optionalen Schritte M9 und M10, erfolgt eine sprachbasierte Steuerung des MR Scanners 1, bei welcher Sprachbefehle mit einer begrenzten Auswahl an sprachlichen Äußerungen, die in der Befehlsdatenbank 40 abgelegt sind, verknüpft sind. Dies ist im Zusammenhang mit MR Systemen besonders vorteilhaft, da es sich bei den sprachlichen Äußerungen oftmals um medizinischtechnischen Jargon handelt. Durch die Bereitstellung der Äußerungen in einer Datenbank wird zudem eine Anpassung an Kundenwünsche erleichtert, da die Äußerungen, die mit einem bestimmten Sprachbefehl verknüpft sind ohne Weiteres geändert werden können. Beispielsweise kann der Kunde bzw. das Bedienpersonal selbst die Äußerungen zu einem Sprachbefehl umbenennen.

Die optionalen Schritte M9 und M10 verbessern die Zuverlässigkeit der Steuerung weiter, indem ein durch maschinelles Lernen trainierter Algorithmus eingesetzt wird, um die erfasste Äußerung zu erkennen. Der Algorithmus kann beispielsweise durch eine Vielzahl von Äußerungen, die von verschiedenen Personen gesprochen wurden und die beispielsweise zusammen mit verschiedenen MR typischen Hintergrundgeräuschen aufgenommen wurden, trainiert worden sein.

Obwohl die Erfindung im Detail durch die Ausführungsbeispiele näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Verfahren (M) zur Steuerung eines MR Scanners (1), aufweisend folgende Schritte:
Erfassen (M1) einer sprachlichen Äußerung (E2) mittels einer akustischen Eingabevorrichtung (2);
Erzeugen (M2) eines Sprachdatenstroms aus der erfassten sprachlichen Äußerung (E2);
Ermitteln (M3) eines aktuellen Systemzustands (S1) des MR Scanners (1);
Erstellen (M4) einer Befehlsbibliothek (50), welche eine Auswahl an Sprachbefehlen enthält, denen jeweils eine oder mehrere sprachliche Äußerungen zugeordnet sind, wobei die Auswahl an Sprachbefehlen in Abhängigkeit des aktuellen Systemzustands (S1) des MR Scanners aus einer Befehlsdatenbank (40) geladen wird;
Anwenden (M5) eines Spracherkennungsalgorithmus (41) auf den Sprachdatenstrom, um zu ermittelt, ob dem Sprachdatenstrom eine in der Befehlsbibliothek (50) enthaltene sprachliche Äußerung zuordenbar ist;
Erkennen (M6) der erfassten sprachlichen Äußerung, wenn dem Sprachdatenstrom eine in der Befehlsbibliothek (50) enthaltene sprachliche Äußerung zuordenbar ist;
Ermitteln (M7) eines der erkannten sprachlichen Äußerung zugeordneten Sprachbefehls aus der Befehlsbibliothek (50); und
Erzeugen (M8) eines Steuerkommandos (C1) zur Steuerung des MR Scanners gemäß dem Sprachbefehl.

2. Verfahren (M) nach Anspruch 1, wobei, wenn dem Sprachdatenstrom keine in der Befehlsbibliothek (50) enthaltene sprachliche Äußerung zuordenbar ist, der Sprachdatenstrom einem internetbasierten Online-Spracherkennungsmodul (6) zugeführt wird, wobei das Online-Spracherkennungsmodul (6)eine durch maschinelles Lernen trainierten Erkennungsfunktion (60) auf den Eingangsdatenstrom anwendet (M9) und eine erkannte sprachliche Äußerung als Ausgangsdatenstrom (D6) bereitstellt.

3. Verfahren (M) nach Anspruch 2, wobei überprüft (M10) wird, ob die erkannte sprachliche Äußerung in der Befehlsbibliothek (50) einem Sprachbefehl zugeordnet ist.

4. Verfahren (M) nach einem der voranstehenden Ansprüche, wobei als aktueller Systemzustand (S1) des MR Scanners (1) einer oder mehrerer der folgenden Zustände ermittelt wird:
der MR Scanner befindet sich in einem Standby-Betrieb;
der MR Scanner führt eine vorbestimmte Scansequenz durch;
der MR Scanner befindet sich in einem Pausierzustand, wobei die Durchführung einer vorbestimmten Scansequenz unterbrochen ist;
der MR Scanner ist in einem Vorbereitungszustand zur Durchführung einer vorbestimmten Scansequenz.

5. Nicht-flüchtiger, computerlesbarer Datenspeicher (4), welcher ein Softwareprogramm (41) speichert, das dazu eingerichtet ist, einen Computer (33) zur Durchführung der Schritte eines Verfahrens gemäß einem der voranstehenden Ansprüche zu veranlassen.

6. Datenspeicher (4) nach Anspruch 5, wobei die Befehlsdatenbank (40) auf dem Datenspeicher (4) gespeichert ist.

7. System (100) zur Durchführung eine Magnetresonanztomographie, MR, mit:
einem MR Scanner (1) zur Durchführung einer Scansequenz an einem Patienten;
einer akustischen Eingabevorrichtung (2) zum Erfassen einer sprachlichen Äußerung (E2); und
einer Steuerungsvorrichtung (3) mit einem Eingang (31), welcher mit der Eingabevorrichtung (2) und mit dem MR Scanner (1) verbunden ist, einem Ausgang (32), welcher mit dem MR Scanner (1) verbunden ist;
wobei die Steuerungsvorrichtung (3) dazu eingerichtet ist
- aus der erfassten sprachlichen Äußerung (D2) einen Sprachdatenstrom zu erzeugen;
- einen aktuellen Systemzustand (S1) des MR Scanners (1) zu ermitteln;
- eine Befehlsbibliothek (50) zu erstellen, welche eine Auswahl an Sprachbefehlen enthält, denen jeweils eine oder mehrere sprachliche Äußerungen zugeordnet sind, wobei die Auswahl an Sprachbefehlen in Abhängigkeit des aktuellen Systemzustands (S1) des MR Scanners (1) aus einer Befehlsdatenbank (40) geladen wird,
- einen Spracherkennungsalgorithmus (41) auf den Sprachdatenstrom anzuwenden, um zu ermittelt, ob dem Sprachdatenstrom eine in der Befehlsbibliothek enthaltene sprachliche Äußerung zuordenbar ist,
- die erfasste sprachliche Äußerung (E2) zu erkennen, wenn dem Sprachdatenstrom eine in der Befehlsbibliothek enthaltene sprachliche Äußerung zuordenbar ist,
- einen der erkannten sprachlichen Äußerung zugeordneten Sprachbefehl aus der Befehlsbibliothek zu ermitteln, und
- ein Steuerkommando (C1) zur Steuerung des MR Scanners (1) gemäß dem Sprachbefehl zu erzeugen.

8. System (100) gemäß Anspruch 7, wobei die Eingabevorrichtung (2) ein Mikrophon aufweist.

9. System (100) nach Anspruch 7 oder 8, wobei die Eingabevorrichtung (2) portabel ist und einen Sender (21) zur drahtlosen Datenübertragung aufweist, und wobei der Eingang (31) der Steuerungsvorrichtung (3) einen Empfänger (34) zur drahtlosen Datenübertragung aufweist, der mit dem Sender (21) der Eingabevorrichtung (2) kommuniziert.

10. System (100) nach einem der Ansprüche 7 bis 9, wobei die Steuerungsvorrichtung (3) einen nicht-flüchtigen Datenspeicher (4) gemäß Anspruch 5 oder 6, und einen Prozessor (33) zum Lesen des Datenspeichers (4) aufweist.
